# EUROPEAN PATENT APPLICATION

(11) **EP 4 253 955 A1**
(43) Date of publication of application: **04.10.2023**
(21) Application number: 22165851.1
(22) Date of filing: 31.03.2022
(51) Int. Cl.: G01N 33/50, G01N 33/574

(54) **EXTRACELLULAR VESICLE POPULATIONS IN THE DIAGNOSIS OF CANCER**

(71) Applicant: Albert-Ludwigs-Universität Freiburg, 79098 Freiburg (DE)
(72) Inventor: NAZARENKO, Irina, 79286 Glottertal (DE); WILHELM, Konrad, 79110 Freiburg (DE); SCHULTZE-SEEMANN, Wolfgang, 79249 Freiburg (DE); PANIUSHKINA, Liliia, 79108 Freiburg (DE)
(74) Representative: Maiwald GmbH

(57) **Abstract**

The present invention is concerned with (i) an *in vitro* method of diagnosing prostate cancer (PCa) and/or a stage thereof in a subject suspected to or known to suffer from PCa, comprising the steps as disclosed herein; (ii) the use of a characteristic of extracellular vesicles (EVs) in a method of diagnosing cancer and/or a stage thereof, wherein the characteristic of the EVs is selected from the group consisting of the size, the density, the origin and combinations thereof; and (iii) a method of establishing a method of diagnosing cancer and/or a stage thereof, wherein the method of establishing comprises a step of analyzing a characteristic of extracellular vesicles (EVs), wherein the characteristic of the EVs is selected from the group consisting of the size, the density, the origin, and combinations thereof.

## Description

### FIELD OF THE INVENTION

The present invention is in the field of diagnosing cancer and/or stages thereof using extracellular vesicles (EVs). More specifically, the present invention is *inter alia* directed to an *in vitro* method of diagnosing prostate cancer (PCa) and/or a stage thereof in a subject suspected to or known to suffer from PCa, comprising the steps as described herein. The present invention is further directed to the use of a characteristic of extracellular vesicles (EVs) in a method of diagnosing cancer and/or a stage thereof, wherein the characteristic of the EVs is selected from the group consisting of the size, the density, the origin and combinations thereof. Further, the present invention is directed to a method of establishing a method of diagnosing cancer and/or a stage thereof, wherein the method of establishing comprises a step of analyzing a characteristic of extracellular vesicles (EVs), wherein the characteristic of the EVs is selected from the group consisting of the size, the density, the origin, and combinations thereof.

### BACKGROUND OF THE INVENTION

Many currently employed cancer diagnosis methods including methods for diagnosing prostate cancer (PCa) are based on needle biopsies to obtain tumor tissue (typically several samples of tumor tissue per patient), which is subsequently analyzed *in vitro* for cancer markers. Taking PCa as example, such invasive biopsies have the drawbacks of resulting in pain, bleeding, sexual dysfunction, frequency and urgency of urination, acute urinary retention or even life-threatening septicemia (Raaijmakers E., et al.; "Complication rates and risk factors of 5802 transrectal ultrasound-guided sextant biopsies of the prostate within a population-based screening program", Urology. 2002; 60: 826-30).

Furthermore, early detection of therapy-resistance is a maintaining problem for the successful treatment of many cancer types. In case of PCa, a portion of patients after receiving the androgen deprivation therapy (ADT), recognized as a main stream for the treatment of advanced prostate cancer, become resistant. So the PCa progress to castration-resistant prostate cancer (CRPC). These patients receive mostly the second-line hormonal therapies including abiraterone acetate (AA) and enzalutamide. Both of them prolong overall survival in patients with CRPC, especially metastatic CRPC. However, a possibility to detect resistance to the second-line therapy is currently missing. For such patients becoming resistant to abiraterone or enzalutamide only an increase of the tumor mass is currently an indicator. New diagnostic approaches are urgently needed for the early detection of therapy resistance especially on the late stages of the disease, where each day of wrong therapy costs human life.

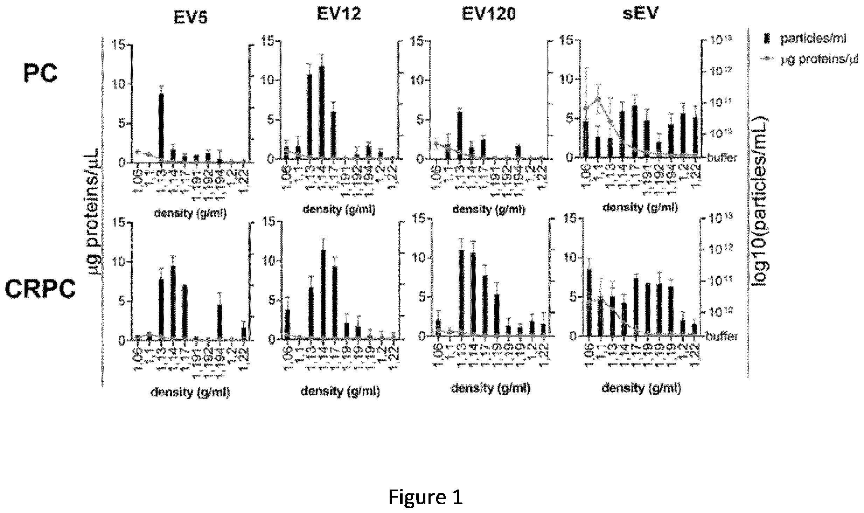

Over the past years, an alternative approach to a needle biopsy has been developed, which is typically referred to as liquid biopsy. Body fluids such as in particular blood (but also e.g. urine, cerebrospinal fluid and seminal plasma) serve as source for the biological markers to be detected and analyzed. When it comes to blood, at least the following components are generally of interest as biological markers for cancer including PCa: cell-free DNA, circulating tumor cells and tumor-derived extracellular vesicles (see Figure 1 of Pang et al.,; "Extracellular vesicles: the next generation of biomarkers for liquid biopsy-based prostate cancer diagnosis", Theranostics. 2020; 10(5):2309-2326).

Tumor-derived extracellular vesicles (EVs) are present in the blood together with EVs derived from other cell types, i.e. the blood comprises EVs from different origins. On a general level, EVs are particles of a size ranging from about 30 nm to about 1000 nm released from different types of cells that are delimited by a lipid bilayer and cannot replicate. EVs play a role in intercellular communication and modulation of target cells. EVs can transport different cargos including proteins, RNAs of different types, DNA fragments and lipids. Furthermore, it seems that EVs can regulate physiological processes and tumor-derived EVs mediate systemic dissemination of various cancers (Introduction-section of Pang et al.; *supra*)*.*

Table 2 of Pang et al.; *supra,* shows a list of putative PCa markers present in EVs for clinical diagnosis such as e.g. CK18, PSA or PTEN in PCa-derived EVs from plasma. Further PCa markers are shown in Table 1 of Akoto and Saini; "Role of exosomes in prostate cancer metastasis", Int. J. Mol. Sci. 2021; 22: 3528, where the EVs are referred to as exosomes. As can be derived from Table 1 of Akoto and Saini, *supra,* specific micro RNAs, long non-coding RNAs, proteins and lipids have been identified in tumor-derived EVs from different body fluids (referred to as sources) as markers for PCa.

Thus, in particular tumor-derived EVs have emerged as carrying markers suitable for cancer, in particular PCa diagnosis, prediction and therapy. There is, however, a need for further markers derived from EVs in PCa and in cancer in general.

### OBJECTS AND SUMMARY OF THE INVENTION

The present inventors have surprisingly found that EVs of a particular size and a particular density can be used to obtain reliable results in a method of diagnosing cancer. For a method of diagnosing PCa and/or a stage thereof (including hormone-independent castration-resistant prostate cancer (CRPC) and in particular therapy-resistant CRPC), the inventors have surprisingly found that the particular size of EVs is about 150 nm to about 400 nm and the particular density of EVs is from about 1.10 g/ml to about 1.17 g/ml.

The present inventors have furthermore surprisingly found that the origin of the EVs serves as a parameter that can be relied upon in a method of diagnosing cancer. For a method of diagnosing PCa and/or a stage thereof, the inventors found that not only EVs derived from the prostate cancer cells are of interest but that rather a combination of EVs of different origin including in particular EVs derived from (i) monocytes/ macrophages, (ii) T-cells, (iii) B-cells and (iv) platelets serve as diagnostic markers as the levels of corresponding surface protein markers indicative of (i) monocytes/ macrophages, (ii) T-cells and (iiii) B-cells differ, in particular increase, from benign prostate hyperplasia (BPH) to malignant prostate cancer (PCa) and to therapy-resistant CRPC. In this respect, the surface protein markers CD14 (indicating a monocyte/ macrophage-origin), CD4 and CD49e (indicating a T-cell-origin), and CD19 (indicating a B-cell origin) were identified by the inventors to be particularly relevant as their levels differed in a statistically significant manner in particular in castration-resistant PCa compared to BPH.

The present inventors have also surprisingly found that nucleic acid cargo of the EVs of a particular size and a particular density can be indicative of a cancer. Thus, for a method of diagnosing PCa and/or a stage thereof, the inventors have identified in the EVs of the particular size and the particular density as outlined above that an RNA indicative of PCa (specifically the RNA splice variant V7 of the androgen receptor) can be used as distinguishing RNA cargo, whereas also the DNA showed differences between the stages hormone-sensitive PCa and therapy-resistant CRPC.

In view of the above, the present application is directed in the first aspect to an *in vitro* method of diagnosing prostate cancer (PCa) and/or a stage thereof in a subject suspected to or known to suffer from PCa, comprising the steps of:
a) providing a plasma sample from the subject;
b) isolating from the plasma sample provided in step a) extracellular vesicles (EVs) with a size ranging from about 50 to about 400 nm and a density of about 1.10 g/ml to about 1.17 g/ml;
c) determining the levels of (i) at least one marker indicating a monocyte/ macrophage-origin, (ii) at least one marker indicating a T-cell-origin and (iii) at least one marker indicating a B-cell origin of the EVs isolated in step b);
d) determining the RNA copy number of an androgen receptor splice variant of the EVs isolated in step b); and
e) assigning PCa and/or a stage thereof to the subject, wherein, when compared to the levels of EVs of a subject with benign prostatic hyperplasia,
   (i) a higher copy number of an androgen receptor splice variant RNA determined in step d) is indicative of PCa; and
   (ii) a higher level of all of (i) to (iii) determined in step c); and a higher copy number of an androgen receptor splice variant RNA determined in step d) is indicative of therapy-resistant CRPC.

In an embodiment thereof, the stage of PCa is therapy-resistant CRPC.

In an embodiment of the first aspect, the at least one marker indicating a monocyte/ macrophage-origin is selected from the group consisting of CD14, CD142/Thromboplastin, and combinations thereof. In another embodiment of the first aspect, the at least one marker indicating a T-cell origin is selected from the group consisting of CD3, CD4, CD8, CD2, CD69, CD45/PTPRC, CD86/FUN-1, CD49e/Integrin Alpha-F (alternatively referred to herein as CD49e), and combinations thereof; preferably from the group consisting of CD3, CD4, CD2, CD69, CD45/PTPRC, CD31/PECAM1, CD56/NCAM1, CD86/FUN-1, CD49e/Integrin Alpha-F, and combinations thereof. In another embodiment of the first aspect, the at least one marker indicating B-cell origin is selected from the group consisting of CD11c/lntegrin Alpha X, CD20/LEU-16, CD49e, CD25/IL2RA, CD19/B4 (alternatively referred to herein as CD19), CD45/PTPRC, CD40/TNFRSF5, CD40/TNFRSF5, CD24, and combinations thereof; preferably from the group consisting of CD11c/lntegrin Alpha X, CD49e, CD25/IL2RA, CD19/B4 (alternatively referred to herein as CD19), CD45/PTPRC, CD40/TNFRSF5, CD40/TNFRSF5, CD24, and combinations thereof.

In an embodiment of the first aspect, the levels of (i) at least one marker indicating a monocyte/ macrophage-origin, (ii) at least one marker indicating a T-cell-origin, (iii) at least one marker indicating a B-cell origin, and (iv) at least one marker indicating a platelet origin of the EVs isolated in step b) are determined in step c); and PCa and/or a stage thereof is assigned in step e), wherein, when compared to the levels of EVs of a subject with benign prostatic hyperplasia,
(i) a higher copy number of an androgen receptor splice variant RNA determined in step d) is indicative of PCa;
(ii) a higher level of all of (i) to (iv) determined in step c); and a higher copy number of an androgen receptor splice variant RNA determined in step d) is indicative of therapy resistant CRPC.

In an embodiment thereof, the at least one marker indicating a platelet-origin is selected from the group consisting of CD41b, CD42a, CD142/Thromboplastin, CD1c/BDCA1-1, CD209/lintegrin Alpha-X, CD31/PECAM1, CD62P/Selectin P, and combinations thereof.

In an embodiment of the first aspect, the levels of (i) at least one marker indicating a monocyte/ macrophage-origin, (ii) at least one marker indicating a T-cell-origin, (iii) at least one marker indicating a B-cell origin, (iv) at least one marker indicating a platelet origin, and (v) at least one marker indicating a cancer origin of the EVs isolated in step b) are determined in step c); and PCa and/or a stage thereof is assigned in step e), wherein, when compared to the levels of EVs of a subject with benign prostatic hyperplasia,
(i) a higher level of (v) determined in step c); and a higher copy number of an androgen receptor splice variant RNA determined in step d) is indicative of PCa;
(ii) a higher level of all of (i) to (v) determined in step c); and a higher copy number of an androgen receptor splice variant RNA determined in step d) is indicative of therapy resistant CRPC.

In an embodiment thereof, the at least one marker indicating a platelet-origin is selected from the group consisting of CD41b, CD42a, CD142/Thromboplastin, CD1c/BDCA1-1, CD209/integrin Alpha-X, CD31/PECAM1, CD62P/Selectin P, and combinations thereof. In an embodiment thereof, the at least one marker indicating a cancer origin is selected from the group consisting of CD44/Epican, CD326/EPCAM and CD29/ITBB1, and combinations thereof.

In an embodiment of the first aspect, the levels of (i) at least one marker indicating a monocyte/ macrophage-origin, (ii) at least one marker indicating a T-cell-origin, (iii) at least one marker indicating a B-cell origin, (iv) at least one marker indicating a platelet origin, (v) at least one marker indicating a cancer origin, and (vi) at least one marker indicating an endothelial cell origin of the EVs isolated in step b) are determined in step c); and PCa and/or a stage thereof is assigned in step e), wherein, when compared to the levels of EVs of a subject with benign prostatic hyperplasia,
(i) a higher level of (v) determined in step c); and a higher copy number of an androgen receptor splice variant RNA determined in step d) is indicative of PCa;
(ii) a higher level of all of (i) to (vi) determined in step c); and a higher copy number of an androgen receptor splice variant RNA determined in step d) is indicative of therapy resistant CRPC.

In an embodiment thereof, the at least one marker indicating a platelet-origin is selected from the group consisting of CD41b, CD42a, CD142/Thromboplastin, CD1c/BDCA1-1, CD209/integrin Alpha-X, CD31/PECAM1, CD62P/Selectin P, and combinations thereof. In an embodiment thereof, the at least one marker indicating a cancer origin is selected from the group consisting of CD44/Epican, CD326/EPCAM and CD29/ITBB1, and combinations thereof. In an embodiment, the at least one marker indicating an endothelial cell origin is selected from the group consisting of CD146/MCAM, CD31/PECAM1, CD62P/Selectin P, CD142/Thromboplastin, CD105/Endoglin, and combinations thereof.

In a preferred embodiment of the first aspect, the levels of (i) at least CD14 indicating a monocyte/ macrophage-origin, (ii) at least CD4 and CD49e indicating a T-cell-origin and (iii) at least CD19 indicating a B-cell origin of the EVs isolated in step b) are determined in step c); and PCa and/or a stage thereof is assigned in step e), wherein, when compared to the levels of EVs of a subject with benign prostatic hyperplasia,
(i) a higher copy number of an androgen receptor splice variant RNA determined in step d) is indicative of PCa;
(ii) a higher level of CD19 determined in step c); and a higher copy number of an androgen receptor splice variant RNA determined in step d) are indicative of hormone-sensitive PCa; and
(iii) a higher level of CD4, CD14, CD19 and CD49e determined in step c); and a higher copy number of AR-V7 RNA determined in step d) are indicative of therapy resistant CRPC.

In an embodiment thereof, an androgen receptor splice variant is selected from the group consisting of AR-23, AR-V3, AR-V4, AR-V7, AR-VB12, AR-45, AR-V2, AR-V9, AR-V13, AR-V14, AR8, AR-V2, AR-V5, AR-V6, AR-V8, AR-V10, AR-V11, and combinations thereof. In a preferred embodiment, an androgen receptor splice variant is the androgen receptor splice variant V7. Details on the androgen receptor splice variants can e.g. be found in Kanayama et al., "AR Splicing Variants and Resistance to AR Targeting Agents" Cancers 2021, 13, 2563.

In a preferred embodiment of the first aspect, the levels of (i) at least CD14 indicating a monocyte/ macrophage-origin, (ii) at least CD4 and CD49e indicating a T-cell-origin and (iii) at least CD19 indicating a B-cell origin of the EVs isolated in step b) are determined in step c); and PCa and/or a stage thereof is assigned in step e), wherein, when compared to the levels of EVs of a subject with benign prostatic hyperplasia,
(i) a higher copy number of AR-V7 RNA determined in step d) is indicative of PCa;
(ii) a higher level of CD19 determined in step c); and a higher copy number of AR-V7 RNA determined in step d) are indicative of hormone-sensitive PCa; and
(iii) a higher level of CD4, CD14, CD19 and CD49e determined in step c); and a higher copy number of AR-V7 RNA determined in step d) are indicative of therapy resistant CRPC.

In an embodiment thereof, the stage of PCa is hormone-sensitive PCa or therapy-resistant CRPC.

In a further embodiment thereof, (ii) a higher level of CD19 but not of CD4, CD14 and CD49e determined in step c); and a higher copy number of AR-V7 RNA determined in step d) are indicative of hormone-sensitive PCa in step e).

In an embodiment, the size of the EVs isolated in step b) is from about 50 to about 100 nm.

In a preferred embodiment, the size of the EVs isolated in step b) is from about 150 to about 400 nm. In an even more preferred embodiment, the size of the EVs isolated in step b) is from about 150 to about 300 nm.

In yet another embodiment relating to the EVs isolated in step b) in the first aspect and the above embodiments of the first aspect, step b) relates to
b) isolating from the plasma sample provided in step a) extracellular vesicles (EVs) with (i) a size ranging from about 50 to about 100 nm and (ii) a size of about 150 nm to about 400 nm, and a density of about 1.10 g/ml to about 1.17 g/ml.

Accordingly, in this preferred embodiment steps c) and d) then relate to
c) determining the levels of (i) at least one marker indicating a monocyte/ macrophage-origin, (ii) at least one marker indicating a T-cell-origin and (iii) at least one marker indicating a B-cell origin of (i) the EVs with a size ranging from about 50 to about 100 nm and a density of about 1.10 g/ml to about 1.17 g/ml isolated in step b) and/or (ii) the EVs with a size ranging from about 150 to about 400 nm and a density of about 1.10 g/ml to about 1.17 g/ml isolated in step b);
d) determining the RNA copy number of an androgen receptor splice variant of (i) the EVs with a size ranging from about 50 to about 100 nm and a density of about 1.10 g/ml to about 1.17 g/ml isolated in step b) and/or (ii) the EVs with a size ranging from about 150 to about 400 nm and a density of about 1.10 g/ml to about 1.17 g/ml isolated in step b).

It is evident from the above embodiment that two different populations of EVs are isolated, namely a population of small EVs with a size ranging from about 50 to about 100 nm and a population of large EVs with a size ranging from about 150 to about 400, preferably about 150 to about 300 nm.

In another embodiment of the first aspect, the subject known to suffer from PCa has been diagnosed using a needle biopsy.

In another embodiment of the first aspect, the plasma sample from the subject is provided outside the human or animal body.

In yet another embodiment of the first aspect, the EVs are isolated from the plasma sample provided in step (a) by a method comprising at least the steps of (i) centrifuging the sample at a low speed (preferably up to about 5.000xg) at a low temperature (preferably at about 2°C to about 15°C, more preferably at about 4°C) for about 20 to about 60 minutes, preferably about 45 minutes; (ii) ultracentrifuging the supernatant from (i) at a high speed (preferably at more than 100.000xg, more preferably at about 350.000xg; 350.000xg are in particular applied if an SW55 Ti rotor is used; the skilled person is aware that the speed might need to be adapted depending on the rotor that is used and is thus capable of adapting the speed to the rotor that is used) and a low temperature (preferably at about 2°C to 15°C, more preferably at about 4°C) for at least about 30 minutes, preferably for about 60 minutes to about 180 minutes, more preferably for about 120 minutes, using a density gradient medium; and (iii) selecting the fractions at densities of about 1.10 g/ml to about 1.17 g/ml. Preferably, the fractions of about 1.10 g/ml, about 1.14 g/ml and about 1.17 g/ml are selected. Most preferably, the fraction at a density of about 1.17 g/ml is selected. A preferred method of isolating and purifying EVs from plasma for the method of the first aspect is outlined in example 6 of the present application. Alternatively, (i) precipitation, (ii) ultrafiltration, (iii) size-exclusion or ion chromatography, (iv) affinity interactions or (v) microfluidic devices and microchips , (vi) field flow fractionation-based methods including AF4 may be used to isolate and/or purify the EVs as long as EVs of the preferred size of about 150 nm to about 400 nm and the preferred density from about 1.10 g/ml to about 1.17 g/ml are obtained.

In yet another embodiment of the first aspect, the levels of the markers (i) to (iii) in step c) are determined by a FACS method using antibodies directed to the markers of interest. A preferred method of determining the levels for the method of the first aspect is outlined in example 6 of the present application, see the MACSPlex Human Exosome analysis, which allows for an analysis of multiple biomarkers from a low amount of material. It is envisaged to preferably use a multiplex analysis of single EVs. In an embodiment, SP-IRIS or nano-flow cytometry allowing for single EV-analysis is applied.

In yet another embodiment of the first aspect, the RNA copy number of an androgen receptor splice variant (such as AR-V7) is determined by a method comprising at least the steps of (i) isolating the complete RNA from the EVs; (ii) reverse transcribing the RNA into cDNA; and (iii) carrying out a quantitative PCR for the androgen receptor splice variant (such as AR-V7). It can be preferred that the quantitative PCR is droplet digital PCR (ddPCR). When it comes to (i), it can be preferred that the EVs are treated with proteases and nuclease (e.g. to avoid contamination of samples with RNA or DNA bound to EVs from the outside) or other suitable chemicals in order to extract the RNA from the EVs as efficiently and purely as possible. Preferred methods of steps (i) to (iii) for the method of the first aspect are outlined in example 6 of the present application.

Although the inventors could not determine a correlation of the EV number and/or EV size to PCa and stages thereof, it cannot be excluded and is rather likely that such a correlation exists for other cancer types. For this reason, the EV number and/or size may generally be relied upon.

In view of the above, the present application is directed in the second aspect to the use of a characteristic of extracellular vesicles (EVs) in a method of diagnosing cancer and/or a stage thereof, wherein the characteristic of the EVs is selected from the group consisting of the size, the density, the origin and combinations thereof.

In an embodiment, the origin is determined using at least one surface marker indicating a specific cell type, preferably wherein the specific cell type is selected from the group consisting of cancer cells, platelets, stem cells, monocytes/ macrophages, T-cells, B-cells, dendritic cells, endothelial cells, neural cells and stromal cells. It is understood that at least one surface marker for each specific cell type is used. In a preferred embodiment, the at least one surface marker indicating EVs is derived from platelets, stem cells, monocytes/ macrophages, T-cells, B-cells, dendritic cells, endothelial cells, neural cells and stromal cells. Suitable surface markers for specific cell types are *inter alia* the following: CD44/Epica, CD326/EPCAM, and CD29/ITGB1 for cancer cells; CD41b, CD42a, CD142/Thromboplastin, CD1c/BDCA1-1, CD209/lintegrin Alpha-X, CD31/PECAM1, CD62P/Selectin P for platelets; CD29/ITGB1, CD105/Endoglin, CD24, and CD133/1 for stem cells; CD14, and CD142/Thromboplastin for monocytes/ macrophages; CD3, CD4, CD8, CD2, CD69, CD45/PTPRC, CD86/FUN-1, CD49e for T-cells; CD11c/lntegrin Alpha X, CD20/LEU-16, CD49e, CD25/IL2RA, CD19, CD45/PTPRC, CD40/TNFRSF5, CD40/TNFRSF5, and CD24 for B-cells; CD1c/BDCA-1, CD209/CD-SIGN, and CD11c/lntegrin Alpha-X for dendritic cells; CD146MCAM, CD31/PECAM1, CD62P/Selectin P, CD142/Thromboplastin, and CD105/Endoglin for endothelial cells; MCSP, ROR1 and S SEA-4 for neural cells;α-smooth muscle actin (a-SMA), fibroblast activation protein (FAP), S100A4, platelet-derived growth factor receptors (PDGFRα/β), and vimentin SMA-alpha for cancer-associated stromal fibroblasts and so called cancer-associated fibroblasts (CAFs); and CD146 for normal stromal cells.

In an embodiment of the second aspect, the EVs are derived from a plasma sample of a subject.

In another preferred embodiment of the second aspect, the characteristic of the EVs is at least a combination of the size, the density and the origin.

In a preferred embodiment of the second aspect, the above characteristic of the EVs is combined with a biomarker of EVs as further characteristic of EVs. Preferably, the biomarker of EVs is selected from at least one biomarker selected from the group consisting of a protein, RNA, DNA, an epigenetic marker, a lipid and combinations thereof. The RNA can in particular be a splice-variant, preferably a splice-variant linked to cancer. The DNA can in particular be a DNA fragment comprising at least one oncogenic mutation. The epigenetic marker can in particular comprise an acetylation and methylation of DNA and/or histones.

In another preferred embodiment of the second aspect, the characteristic of the EVs is at least a combination of the size, the density, the origin and a biomarker. It can be preferred that the biomarker is at least one biomarker selected from the group consisting of a protein, RNA, DNA, an epigenetic marker, a lipid and combinations thereof, preferably RNA. It can also be preferred that the biomarker is a combination of at least two biomarkers selected from the group consisting of a protein, RNA, DNA, an epigenetic marker and a lipid, preferably from the group consisting of a protein, RNA and DNA.

In view of the above, the present application is directed in the third aspect to a method of establishing a method of diagnosing cancer and/or a stage thereof, wherein the method of establishing comprises a step of analyzing a characteristic of extracellular vesicles (EVs), wherein the characteristic of the EVs is selected from the group consisting of the size, the density, the origin and combinations thereof.

In an embodiment, the origin is determined using at least one surface marker indicating a specific cell type, preferably wherein the specific cell type is selected from the group consisting of cancer cells, platelets, stem cells, monocytes/ macrophages, T-cells, B-cells, dendritic cells, endothelial cells, neural cells and stromal cells. It is understood that at least one surface marker for each specific cell type is used. In a preferred embodiment, the at least one surface marker indicating EVs is derived from platelets, stem cells, monocytes/ macrophages, T-cells, B-cells, dendritic cells, endothelial cells, neural cells and stromal cells. Suitable surface markers for specific cell types are *inter alia* the following: CD44/Epica, CD326/EPCAM, and CD29/ITGB1 for cancer cells; CD41b, CD42a, CD142/Thromboplastin, CD1c/BDCA1-1, CD209/lintegrin Alpha-X, CD31/PECAM1, CD62P/Selectin P for platelets; CD29/ITGB1, CD105/Endoglin, CD24, and CD133/1 for stem cells; CD14, and CD142/Thromboplastin for monocytes/ macrophages; CD3, CD4, CD8, CD2, CD69, CD45/PTPRC, CD86/FUN-1, CD49e for T-cells; CD11c/lntegrin Alpha X, CD20/LEU-16, CD49e, CD25/IL2RA, CD19, CD45/PTPRC, CD40/TNFRSF5, CD40/TNFRSF5, and CD24 for B-cells; CD1c/BDCA-1, CD209/CD-SIGN, and CD11c/lntegrin Alpha-X for dendritic cells; CD146MCAM, CD31/PECAM1, CD62P/Selectin P, CD142/Thromboplastin, and CD105/Endoglin for endothelial cells; MCSP, ROR1 and S SEA-4 for neural cells; α-smooth muscle actin (a-SMA), fibroblast activation protein (FAP), S100A4, platelet-derived growth factor receptors (PDGFRα/β), and vimentin SMA-alpha for cancer-associated stromal fibroblasts and so called cancer-associated fibroblasts (CAFs); and CD146 for normal stromal cells.

In an embodiment of the third aspect, a biomarker as further characteristic of EVs is analyzed. Preferably, the biomarker of EVs is selected from at least one biomarker selected from the group consisting of a protein, RNA, DNA, an epigenetic marker, a lipid and combinations thereof. The RNA can in particular be a splice-variant, preferably a splice-variant linked to cancer. The DNA can in particular be a DNA fragment comprising at least one oncogenic mutation. The epigenetic marker can in particular comprise an acetylation and methylation of DNA and/or histones.

In a preferred embodiment of the third aspect, the EVs are derived from (i) a plasma sample of a subject not suffering from cancer, (ii) a plasma sample from a subject suffering from cancer and/or (iii) a plasma sample from a subject suffering from a stage of the cancer. A size and/or density and/or origin and/or, if analyzed, a biomarker is then assigned to the EVs derived from (i) to (iii) in this embodiment. Preferably, a difference between the size and/or density and/or origin and/or, if analyzed, the biomarker of EVs is determined between the EVs derived from (i) to (iii) in this embodiment. Accordingly, a difference between at least one characteristic is determined between the different subjects, namely the subject not suffering from cancer, the subject suffering from cancer and/or the subject suffering from a stage of the cancer. It is preferred that this determination is carried out by an AI-based algorithm. In another preferred embodiment, the size and/or density and/or origin and/or, if analyzed, biomarker of EVs exhibiting a difference between the EVs derived from (i) to (iii) is a marker to be used in a method of diagnosing cancer and/or a stage thereof.

### DESCRIPTION OF THE FIGURES

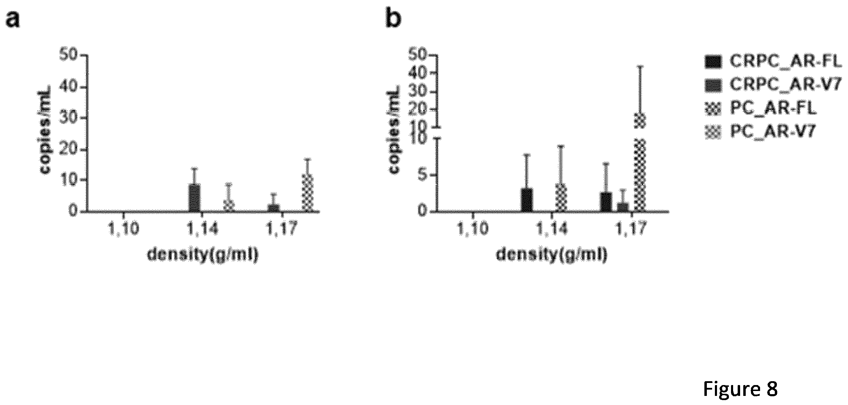

Figure 1. Analysis of particles measured by NTA and proteins detected by MicroBCA for PCa and CRPC-isolated plasma samples. For PCa (top) and therapy-resistant CRPC (bottom), the four fractions (i) to (iv) are shown as EV5, EV12, EV120 and sEV, wherein the protein amount is given in the left y-axis as µg protein/µl and the particle numbers are given in the right y-axis as log10 (particles/ml). Each x-axis shows the density gradient fractions (g/ml) of each of fractions (i) to (iv).

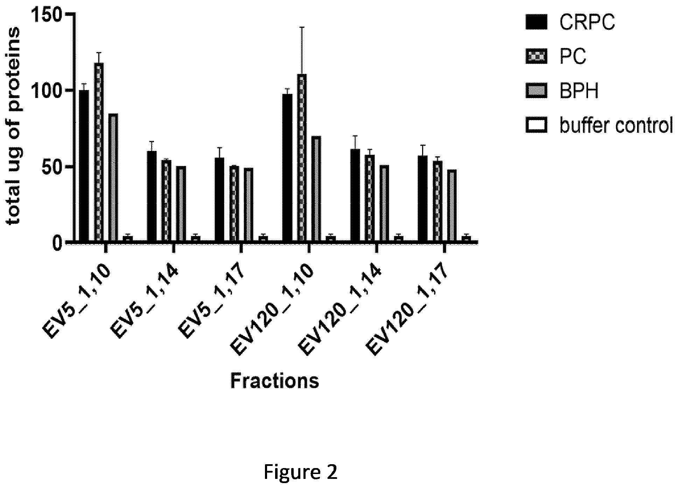

Figure 2. Total protein measurements of the samples isolated from BPH, PCa and CRPC plasma before MACSPlex analysis. The total protein amounts were analyzed and is depicted as µg protein in the y-axis with the three different fractions 1.10, 1.14 and 1.17 shown for the EV5 fraction (large EVs, left side) and the EV120 fraction (small EVs, right side) on the x-axis.

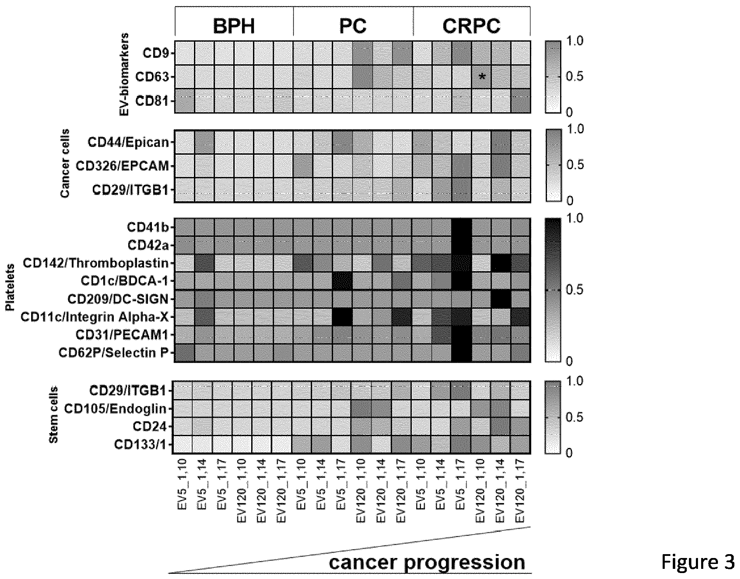

Figure 3. Prostate cancer progression clustering of EVs, cancer cells, platelets and stem cells surface biomarkers. The clustering analysis of EV- (panel 1), cancer cells- (panel 2), platelets- (panel 3) and stem cells- (panel 4) associated molecules was carried out using MACSPlex Human exosomes kits using beads-assistant flow cytometry in 1.10, 1.14 and 1.17-fractions of EV5 and EV120 fractions isolated from plasma of BPH, PCa and CRPC patients.

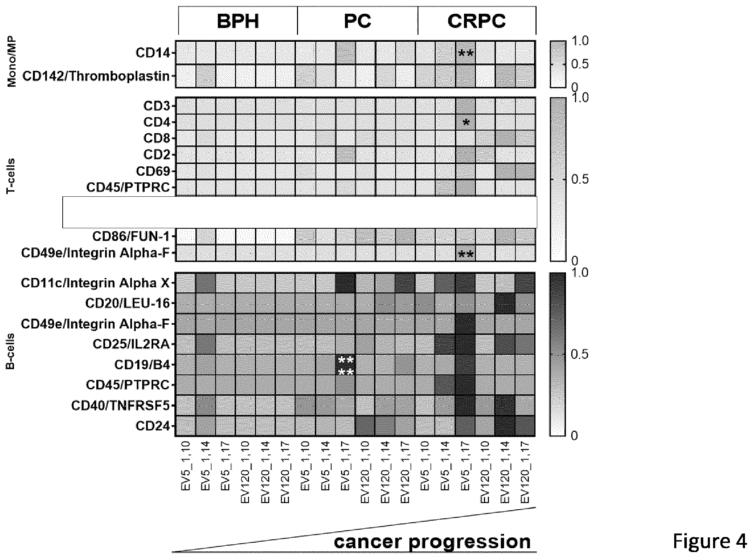

Figure 4. Prostate cancer progression clustering of monocytes, MP, T- and B- cells, surface biomarkers. The clustering analysis of monocytes/macrophages- (panel 1), T-cells- (panel 2), and B-cells - (panel 3) associated molecules was carried out using MACSPlex Human exosomes kits using beads-assistant flow cytometry in 1.10, 1.14 and 1.17-fractions of EV5 and EV120 fractions isolated from plasma of BPH, PCa and CRPC patients.

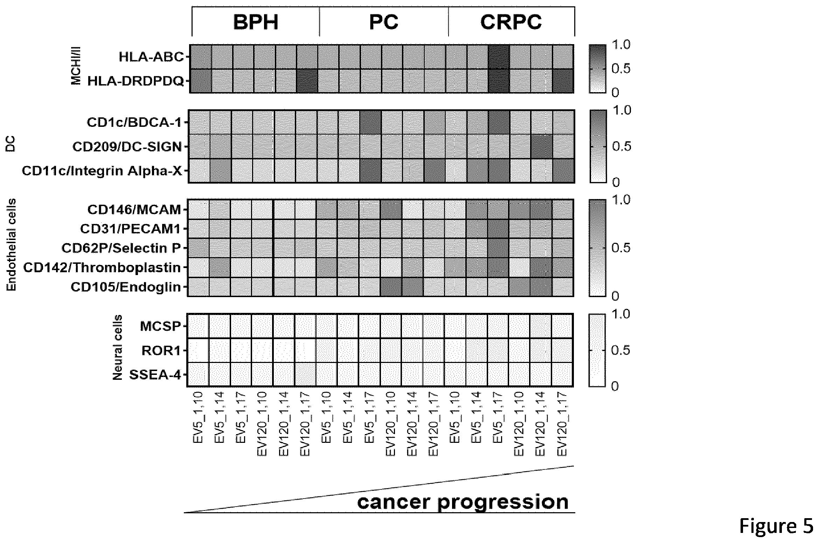

Figure 5. Prostate cancer progression clustering of MCHI/II, DC, endothelial and neural cells surface biomarkers. The clustering analysis of MCHI/II- (panel 1), DC- (panel 2), endothelial cells-(panel 3) and neural cells- (panel 4) associated molecules was carried out using MACSPlex Human exosomes kits using beads-assistant flow cytometry in 1.10, 1.14 and 1.17-fractions of EV5 and EV120 fractions isolated from plasma of BPH, PCa and CRPC patients.

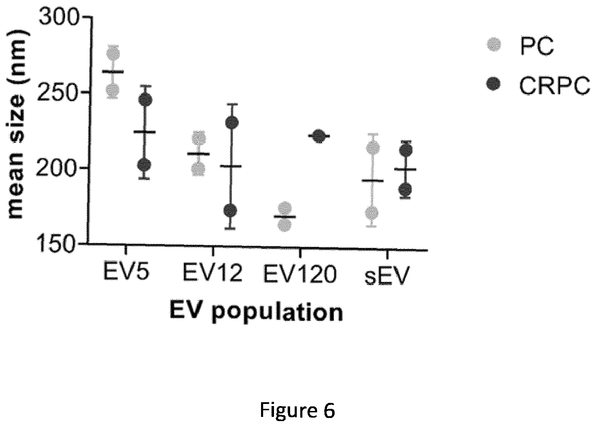

Figure 6. Mean size of fractions (i) to (iv) prior to purification. The mean sizes (in nm) of the EVs in the four different fractions from PCa patients (left) and CRPC patients (right) before density gradient purification are shown.

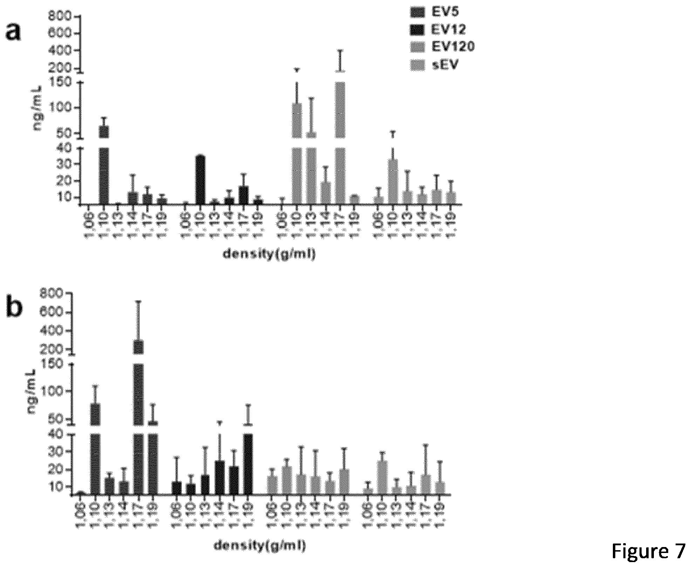

Figure 7. RNA distribution among different EV populations isolated from patient's plasma.
   (a) RNA distribution among different EV populations isolated from PCa patient's plasma. The amount of RNA (in ng/ml) is shown in the y-axis, whereas the x-axis depicts the different fractions of the four initial fractions (i) to (iv) of samples isolated from PCa patients.
   (b) RNA distribution among different EV populations isolated from CRPC patient's plasma. The amount of RNA (in ng/ml) is shown in the y-axis, whereas the x-axis depicts the different fractions of the four initial fractions (i) to (iv) of samples isolated from CRPC patients
Figure 8. Comparison of AR-FL and AR-V7 expression among different EV populations from patient's plasma. The number of copies/ml is shown in the y-axis for AR-FL (dark) and AR-V7 (light) in the PCa and CRPC samples from EV5, large EVs, (a) and EV120, small EVs, (b) fractions.

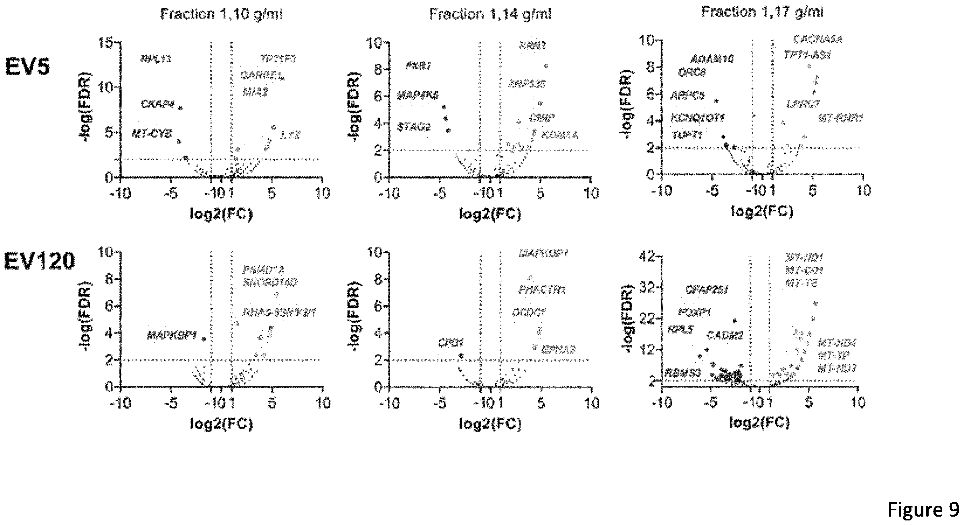

Figure 9. Volcano-plot distribution of significantly different gene enrichment in EV5 and EV120 populations from PCa and CRPC patients' plasma. The distribution of genes significantly expressed in PCa (right side of each plot) and CRPC (left side of each blot) for EV5 and EV120 fractions is shown separately for corresponding fractions at 1.10, 1.14 and 1.17.

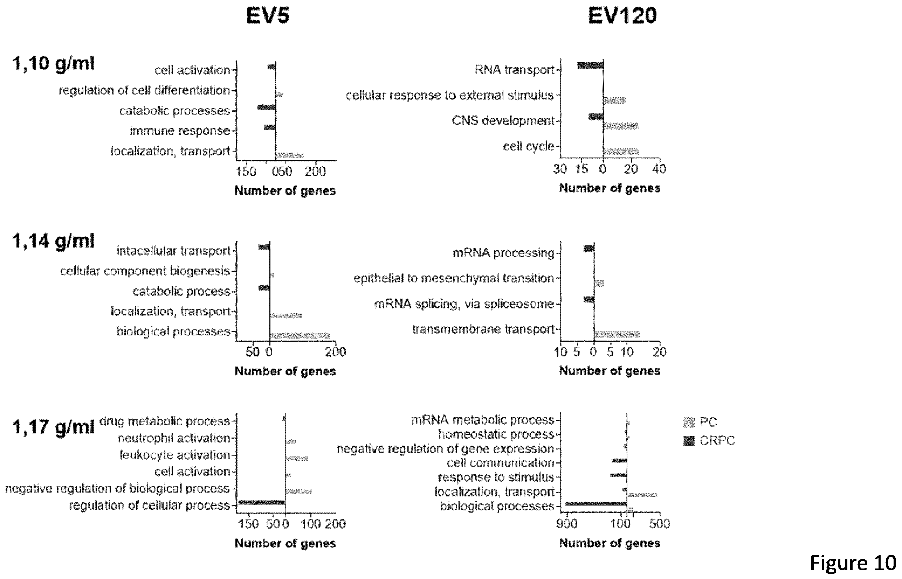

Figure 10. Analysis of biological pathways represented by the significantly different genes detected on EV5 and EV120 fractions from PCa and CRPC patients' plasma. Gene Ontology analysis of genes from PCa (right side of each plot) and CRPC (left side of each plot) in the EV5 and EV120 fractions of the plasma samples.

### DETAILED DESCRIPTION OF THE INVENTION

Before the present invention is described in more detail, the following definitions are introduced.

### 1. Definitions

As used in the specification and the claims, the singular forms of "a" and "an" also include the corresponding plurals unless the context clearly dictates otherwise.

The term "about" in the context of the present invention denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present invention, the term "consisting of" is considered to be a preferred embodiment of the term "comprising". If hereinafter a group is defined to comprise at least a certain number of embodiments, this is also meant to encompass a group which preferably consists of these embodiments only.

The term "benign prostatic hyperplasia" or "BPH" relates to a non-cancerous enlargement of the prostate gland. BPH is the most common benign tumor found in men. As BPH is not a cancer but a benign state, BPH can be used as reference in terms of progression to a cancer state.

The term "prostate cancer" or "PCa" relates to a cancer of the prostate gland in the male reproductive system, which occurs when cells of the prostate transform from normal to malignant, multiply without control and become motile and able to invade into neighboring tissues. As used herein, this term does not indicate a specific stage of the cancer but merely the presence of PCa. Thus, a "prostate cancer" as used herein denotes a prostate cancer which can be classified according to the TNM classification by the International Union Against Cancer (UICC) into stages I to IV and/or according to the Gleason score.

"Hormone-sensitive PCa" relates to PCa that might (if so, it would be metastatic hormone-sensitive PCa) or might not yet have spread past the prostate into the body, but can be treated with hormone therapy, because the cells still need a male hormone (androgen) to grow. The therapy either stops the production or blocks the production of androgens, which is known as castration. It can be regarded as an early stage of PCa. Hormone-sensitive PCa is a stage of PCa.

"Castration-resistant PCa" or "CRPC" relates to PC that typically develops during the treatment of hormone-sensitive PC by hormonal therapy. The tumor will evade this by requiring much lower levels of androgens to progress, which means that the tumor progresses to a later stage of PCa, which is characterized in that withdrawal of androgen levels by a the hormone therapy will no longer stop the growth of the tumor. CRPC is a stage of PCa. "Therapy resistant CRPC" means a stage of PCa that is resistant to abiraterone and enzalutamide therapy (so-called "second line therapy").

The term "extracellular vesicles" or "EVs" relates to particles of a size ranging from about 30 nm to about 1000 nm, which are released from different types of cells and which are delimited by a lipid bilayer and which cannot replicate. EVs can transport different cargos including proteins, nucleic acids including RNAs of different types and DNA fragments, some metabolites and lipids.

The term "size of EVs" means the particle size of the EVs, which is typically the diameter of the EVs in nm. The term "density of EVs" means the weight of the EVs per volume, which is typically expressed as g/ml. The term "origin of EVs" as used herein relates to the cellular source of the EVs or the cells, from which the EVs stem.

A copy number is deemed to be at a "higher level" if elevated more than about 2 fold, about 5 fold or about 10 fold or even more than about 10 fold compared to the corresponding level of EVs of a subject with benign prostatic hyperplasia.

A level of a marker is deemed to be at a "higher level" if elevated more than about 0.5 fold, about 0.6 fold, about 0.7 fold, about 0.8 fold, about 0.9 fold, about 1 fold or even more than about 1 fold, or higher, compared to the corresponding level of EVs of a subject with benign prostatic hyperplasia.

### 2. Findings by the inventors

The present inventors have surprisingly found that EVs of a particular size and a particular density can be used to obtain reliable results in a method of diagnosing PCa (including stages thereof). A particular size of EVs might even be sufficient for a cancer diagnosis. For PCa as described in more detail in the examples section, a separation by size was carried out first, followed by a density separation within each size fraction. The density separation may also be referred to as purification of the EVs of different sizes.

The inventors have surprisingly found that two populations of EVs, namely a population of (i) the size of EVs of about 50 to about 100 nm ("small EVs") and a population of (ii) the size of EVs of about 150 to about 400 nm ("large EVs") and each population of the density of from about 1.10 g/ml to about 1.17 g/ml, most preferably the size of EVs of about 150 nm to about 300 nm and the density of EVs of from about 1.10 g/ml to about 1.17 g/ml result in a fraction of EVs that can be used to obtain reliable results in a method of diagnosing PCa and/or a stage thereof. The size, optionally in combination with the density, might be in the same range for other cancers and/or a stage thereof.

The present inventors have furthermore surprisingly found that the origin of the EVs serves as a parameter that can be relied upon in a method of diagnosing cancer, as set out in detail in the next paragraph. For PCa, this was found for the EVs of a size of about 50 nm to about 400 nm, in particular a size of about 150 nm to 300 nm, and a density of EVs is from about 1.14 g/ml to about 1.17 g/ml, with a density of about 1.17 g/ml being preferred. The origin as parameter that can be relied upon in such a method for other cancers might need to be determined for EVs in the same range.

For a method of diagnosing PCa and/or a stage thereof, the inventors surprisingly found that not only EVs derived from prostate cancer cells are of interest but that in particular EVs derived from other cell types, such as in particular (i) monocytes/ macrophages, (ii) T-cells and (iii) B-cells serve as diagnostic markers as the levels of corresponding surface protein markers indicative of (i) monocytes/ macrophages, (ii) T-cells, (iiii) B-cells, and (iv) platelets increase from benign prostate hyperplasia (BPH) to hormone-sensitive PCa, and from hormone-sensitive PCa to castration-resistant PCa (CRPC) including therapy-resistant CRPC. The surface protein markers CD14 (indicating a monocyte/ macrophage-origin), CD4 and CD49e (indicating a T-cell-origin), and CD19 (indicating a B-cell origin) were identified by the inventors to be particularly relevant as their levels differed in a statistically significant manner in particular in castration-resistant PCa compared to BPH. For other cancers, a different pattern in terms of the origin may emerge, wherein the origin is in particular selected from the group of platelets, stem cells, monocytes/ macrophages, T-cells, B-cells, dendritic cells, endothelial cells, neural cells, stromal cells and combinations thereof, reflecting systemic changes in the body accompanying different stages of the disease.

The present inventors have also surprisingly found that nucleic acid cargo of the EVs of a particular size and a particular density can be indicative of a cancer. Thus, for a method of diagnosing PCa and/or a stage thereof, the inventors have identified in the EVs of the particular size and the particular density as outlined above that an RNA indicative of PCa (e.g. an androgen receptor splice variant RNA and in particular the splice variant V7 of the androgen receptor) can be used as distinguishing RNA cargo. For other cancers, RNA may also be characteristic, in particular RNA specific for the cancer, such as e.g. cancer-characteristic splice variants (e.g. AGR2 splice variants). The DNA fragments in the EVs as biomarker may also be characteristic, in particular DNA fragments comprising oncogenic mutations.

In the following section, particular examples illustrating various embodiments and aspects of the invention are presented. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become readily apparent to those skilled in the art from the foregoing description, accompanying figures and the examples below. All such modifications fall within the scope of the claims as disclosed herein.

### 3. Examples

### Example 1: Characterization of the EV-fractions for the further analysis after isolation and purification of the EVs

In order to characterize the ten fractions obtained after purification of each of the four EV-fractions (i) to (iv), which were obtained after isolation as described in example 6 below, all 40 fractions of a given sample were subjected to determination of the particle density (using nanoparticle tracking analysis) and the amount of protein (using a BCA protein assay). While at least one fraction of the fractions of 1.10, 1.14 and 1.17 over all four different fractions (i) to (iv) for BPH, PCa and CRPC appeared to be suitable for the further analysis, the following fractions were identified as displaying an optimum combination of density and protein concentration, as can be derived from Figure 1: in EV5 [EV-fraction (i)] fraction 1.13 for PCa and fractions 1.13, 1.14 and 1.17 for CRPC; in EV12 [EV-fraction (ii)] fractions 1.13, 1.14 and 1.17 for PCa and for CRPC; in EV120 [EV-fraction (iii)] fractions 1.13 and 1.17 for PCa and fractions 1.13, 1.14, 1.17 and 1.191 for CRPC; and sEV [fraction (iv)] fractions 1.14, 1.17 and 1.191 for PCa and for CRPC (data not shown for BPH).

### Example 2: Analysis of biomarkers indicating the different origins of the EVs

Given that at least one fraction of the fractions of 1.10, 1.14 and 1.17 was deemed to be suitable for a further analysis, these three fractions of the EV5 [EV-fraction (i)] and EV120 [EV-fraction (iii)] were chosen for the present analysis of the EVs with respect to their origins and respective markers. The overall protein amounts (determined using a BCA protein assay) were comparable amongst the different fractions of the BPH, PCa and CRPC samples, see Figure 2. 74 samples including buffer controls in replicates were used. The present analysis was carried out using a MACSPlex Human Exosome analysis in order to determine whether or not EVs from different origins and/or corresponding markers show a different pattern depending on the cancer stage. To this aim, EVs from the following types/origins were analyzed using the markers given in brackets: EV-biomarkers (CD9, CD63, CD81); cancer cells (CD44/Epican, CD326/EPCAM, CD29/ITGB1); plateletes (CD41b, CD42a, CD142/Thromboplastin, CD1c/BDCA-1, CD209/DC-SIGN, CD11c/lntegrin Alpha-X, CD31/PECAM1, CD62P/Selectin P); stem cells (CD29/ITGB1, CD105/Endoglin, CD24, CD133/1); monocytes (Mono)/macrophages (MP) (CD14, CD142/Thromboplastin); T-cells (CD3, CD4, CD8, CD2, CD69, CD45/PTPRC, CD86/FUN-1, CD49a/Integrin Alpha-F); B-cells (CD11c/Integrin Alpha X, CD20/LEU-16, CD49e/Integrin Alpha-F, CD25/IL2RA, CD19/B4, CD45/PTPRC, CD40/TNFRSF5, CD24); MCHI/II (HLA-ABC, HLA-DRDPDQ); DC (CD1c/BDCA-1, CD209/DC-SIGN, CD11c/lntegrin Alpha-X); endothelial cells (CD146/MCAM, CD31/PECAM1, CD62P/Selectin P, CD142/Thromboplastin, CD105/Endoglin); and neural cells (MCSP, ROR1, SSEA-4).

Figures 3 to 5 show the results obtained from the analysis: On a general level, there seems to be a trend in particular for fraction 1.17 from EV5 for higher levels of the biomarkers of EVs derived from platelets, monocytes/macrophages, T-cells and B-cells depending on the cancer progression (from BPH to CRPC). Statistically significant higher levels were obtained for CD63 in fraction 1.10 of EV120 of CRPC (p-value=0.0248, see asterisk in EV-biomarkers in Figure 3), for CD14 in fraction 1.17 of EV5 of CRPC (p-value=0.0032, see asterisks in Mono/MP in Figure 4), for CD4 and CD49e/Integrin Alpha F in fraction 1.17 of EV5 of CRPC (p-values=0.012 and 0.0076, respectively, see asterisks in T-cells in Figure 4), and for CD19/B4 in fraction 1.17 of EV5 of PCa (p-value<0.0001, see asterisks in Figure 4).

Summarizing the MACSPlex analysis results, overexpression of B-cell-associated molecule CD19 was determined in PCa in fraction 1.17 of EV5. Accordingly, CD19 might serve as indicator when BPH turns to PCa and CRPC. Further, the 1.10 fraction of EV120 was significantly enriched in the EV-associated CD63 biomarker in CRPC. Finally, the 1.17 fraction of EV5 was found to comprise higher levels of CD14, CD4 and CD49e indicative of CRPC, indicating a potential monocytes and T-cell activation. These data might be interpreted such that an immune response is activated when prostate cancer is progressing. It is noted that the increased immune cell biomarkers were identified in the EV5 fraction, i.e. the large EVs of a size of about 150 nm to about 600 nm.

### Example 3: No correlation of EV number and EV size to different PCa stages

The number of particles in the four different EV-fractions (EV5, EV12, EV120 and sEV) after isolation but prior to purification by ultracentrifugation was analyzed in the samples from patients suffering from PCa and CRPC in order to determine whether or not the particle number of EVs could be used as marker for the different PCa stages. The number of particles was determined by NTA (as particles/ml) but no correlation to a specific stage was found (data not shown).

The size of particles in the four different EV-fractions (EV5, EV12, EV120 and sEV) after isolation but prior to purification by ultracentrifugation was analyzed in the samples from patients suffering from PCa and CRPC in order to determine whether or not the particle size of EVs could be used as marker for the different PCa stages. The size of particles was determined by ILM but no correlation to a specific stage was found (see Figure 6).

### Example 4: RNA distribution and presence of RNA encoding AR-FL vs. AR-V7

In the next step, total RNA was isolated as described in example 6 from fractions 1.06 to 1.19 of the four EV fractions (i) to (iv), and the RNA concentrations were determined as described in example 6. Normalization of the samples was carried out by volume (normalization by particle number or protein amount was not possible due to heterogeneous samples, data not shown). RNA concentrations in the different fractions of the PCa samples are shown in Figure 7a, whereas RNA concentrations in the different fractions of the CRPC samples are shown in Figure 7b. As can be derived from Figures 7a and b, there is an increase in the RNA concentrations in the fractions derived from EV5 and EV12 (i.e. the large EVs) when going from PCa to CRPC and a decrease in the RNA concentrations in the fractions derived from EV120 (i.e. the smaller EVs) when going from PCa to CRPC. Thus, there seems to be a shift from EV120 in PCa to EV5 and EV12 in CRPC (i.e. from small to large EVs).

Given that most RNA is localized in large EVs (EV5) for CRPC and small EVs (EV120) for PCa, the surface protein distribution was analyzed and it was found that all fractions were CD63 positive. Further, all fractions of PCa samples had a CD9 signal, while the 1.10 and 1.14 fractions of EV5 in CRPC samples were CD9 negative. Interestingly, EV5 fractions from both PCa and CRPC did not contain CD3, whereas CD3 appears at the 1.10 and 1.14 fractions of EV120 in PCa samples and the 1.10 and 1.17 fractions of EV120 of CRPC samples. All fractions were CD41 positive, whereas the distribution of PSMA was different for PCa and CRPC samples: fraction 1.17 of EV5 and fractions 1.14 to 1.17 of EV120 in PCa samples were negative to PSMA, while most of the CRPC fractions were PSMA positive (with the exception of the 1.17 fraction of EV120).

Finally, it was of interest whether the RNA distribution among EV population from PCa and CRPC samples is linked to the expression of the androgen receptor (AR) full length (AR-FL) and its splice variant V7 (AR-V7). To this aim, plasma samples from high Gleason score (6-8) PCa patients and patients with confirmed CRPC were obtained, and small and large EVs were isolated and purified as described herein. Subsequently, total RNA was isolated and a cDNA synthesis was carried out as described in example 6. Finally, a ddPCR for the quantitative detection of AR-FL and AR-V7 was carried out as described in example 6. The results are shown in Figures 8a (EV5, i.e. large EVs) and 8b (EV120, i.e. small EVs): Remarkably, in large EVs (EV5), no full length AR mRNA was detected, but rather the AR-V7 variant. However, the AR-V7 variant was present in both, the PCa and CRPC samples, indicating that some of the PCa samples analyzed may develop the resistance to enzalutamide and abiraterone. No AR-V7 was detected in small vesicles (EV120) in PCa samples, showing that the large EVs (EV5) can be more indicative for the potential resistance to the therapy.

### Example 5: Gene expression patterns determined on the basis of the total RNA in the different samples

Next-Generation Sequencing (NGS) via MACE (Massive Analysis of cDNA Ends) analysis was used to identify the gene expression patterns in the different samples and the total number of genes that were found was 11333. The distribution of significantly (p-value <0,05, FC>2) expressed genes in 1.10, 1.14 and 1.17 fractions of EV5 and EV120 fractions of PCa and CRPC samples is shown on Figure 9. In general, the 1.17 fraction from both EV5 and EV120 was the fraction mostly enriched with significantly different genes for PCa vs. CRPC comparison. Interestingly, the EV120 fraction from PCa samples contained mitochondrial genes while the corresponding fraction derived from CRPC did not. To highlight the functions behind those genes, a Gene Ontology (GO) analysis of biological pathways was performed with the results shown in Figure 10. The GO analysis of genes from PCa and CRPC plasma samples showed EV size- and density-dependent diversities. The genes from EV5 population in CRPC case have a catabolic function, while in PCa they mostly carry localization and transport functions. Importantly, immune response genes in CRPC already appear in the 1.10 fraction, while in PCa leukocytes and neutrophil activation genes in the 1.17 fraction. Another important biological pathway involved in drug metabolism is specifically present in EV5 in the 1,17 fraction from CRPC samples. The genes found in the EV120 fraction from CRPC patients are mainly related to RNA transport, mRNA processing and mRNA splicing. The genes from the EV120 fraction of the PCa samples relate to a cellular response to an external stimulus, epithelial to mesenchymal transition and mRNA metabolic processes.

Furthermore, an analysis of so-called oncogenic signatures (taken from the MSigDB - GSEA database) was carried out in the different samples and it was found that the 1.10 fraction of EV5 and EV120 from PCa samples did not contain any oncogenic signatures while the corresponding CRPC fractions contained such oncogenic signatures in a highly enriched manner. In other fractions, some of the oncogenic signatures were similar in EV5 and EV120 fractions of PCa and CRPC. Interestingly, KRAS mutations were specifically present in fraction 1.14 of EV5 and EV120 in PCa samples. The signature P53 DN.V1 UP was specific to the 1.10 fraction of EV5 and EV120 in CRPC samples. The ESC J1 UP LATE.V1 UP signature was associated with fraction 1.14 in EV5 and EV120 of CRPC. One of the most interesting set JNK DN.V1 DN known to decrease apoptosis was detected in fraction 1.17 of EV120 of PCa samples and 1.10 of CRPC.

Overall, the total enrichment of oncogenic signatures in EV populations is higher in CRPC samples (18 against 7). The 1.10 fraction was of particular interest in the CRPC samples while it did not have oncogenic signatures in the PCa samples. The oncogenic sets that were detected in CRPC samples despite EV size were P53 DN.V1 UP and ESC J1 UP LATE.V1 UP. The gene sets found in EV5 and EV120 populations at fraction 1.14 of PCa samples were KRAS.BREAST UP.V1 UP and KRAS.AMP.LUNG UP.V1 UP. The oncogenic signature JNK DN.V1 DN was detected in both EV120 fractions of PCa and CRPC patient samples with a tendency to fraction 1.10 for CRPC and fraction 1.14 for PCa. The distribution of oncogenic signatures among large and small EV fractions isolated from PCa and CRPC plasma samples is different. The enrichment of fractions at 1.10 in CRPC samples might indicate a formation of HDL-LDL-EV complexes that are not present in PCa plasma. In other words, the activation of small molecules transfer together with HDL increase while catabolic activity going up might indicate prostate cancer progression. Importantly, the oncogenic sets differentially distributed among EV populations in PCa and CRPC plasma samples suppress antigen representation and downregulate apoptosis. Those important pathways might be a hint to the mechanisms of immune response formation during prostate cancer progression. Finally, direct and indirect AR pathway suppression could be found, resulting in a decrease of hormone receptor formation that could be monitored by AR-FL and AR-V7 detection in large and small EV populations.

### Example 6: Material and Methods

Isolation and purification of EVs from patient's plasma: 1 ml blood from patients with BPH or patients diagnosed with PCa or therapy-resistant CRPC (if reference in the examples is made to "CRPC", this is to be understood as reference to "therapy resistant CRPC") was collected into ACD tubes (ADT) at the Urology department of the Medical Center University of Freiburg. The tubes were transported in a vertical rack to the laboratory no later than in 1 hour after collection. Each sample was subject to hemolysis and two centrifugation steps at 2.500xg for 15min at RT prior to the isolation of the EV-fractions described in the following. Four different EV-fractions were gained for each sample: (i) EV5 in 100 µl PBS after a centrifugation at 5.000xg for 45min at 4°C; (ii) EV12 in 100 µl PBS after a further centrifugation at 12.000xg for 45min at 4°C; (iii) EV120 in 100 µl PBS after a further ultracentrifugation at 120.000xg for 60min at 4°C; and (iv) sEV in up to 200 µl after concentration by ultrafiltration columns, Amicon (cut-off 10 kDa) or X-Spinner (cut-off 100 kDa), while the latter were more efficient. At this stage, each fraction comprised EVs, lipoproteins and plasma proteins. All EV-fractions (i) to (iv) were subsequently subjected to a purification in order to remove other components than the EVs by a density gradient centrifugation at 350.000xg for 60min at 4°C (in an SW55 Ti rotor) using an iodixanol-based density gradient medium for the isolation of EVs (OptiPrep). For each purification, 10 fractions were obtained and are referred to herein as starting from the lowest density (g/ml): 1.06; 1.1; 1.13; 1.14; 1.17; 1.191; 1.192; 1.194; 1.2; and 1.22.

NTA (Nanoparticle Tracking Analysis): A ZetaView nanoparticle tracking videomicroscope was used. For the analysis, all samples were diluted in 0,1XPBS (0.22 mm-filtered) to a final volume of 1 ml. For each measurement, scanning of 11 positions and capturing 30 frames per position with a medium speed for 5 cycles was performed. The capturing settings were as follows: autofocus; camera sensitivity for all samples: 85.0; shutter: 70; scattering intensity: 4.0; cell temperature: 25°C. The videos were analysed by the in-build ZetaView Software with the analysis parameters: maximum particle size: 1000, minimum particle size 10, minimum particle brightness: 20.

ILM (Interferometric Light Microscopy): The concentration of particles was measured by the ILM method using the VideoDrop instrument (Myriade, Paris, France). For each measurement, 10 videos of 100 frames (acquisition time 7.15ms, exposure time 0.9s) were recorded with a minimum of 100 tracked particles. The videos were analysed using Qvir (2.5.3) software with the following processing settings: detection threshold 4.2, remove macroparticles function 'activated' (min radius 10; min hot pixel 150), viscosity 1.05 mPa.s and temperature 293K. The proof of concept was realised with size-calibrated polystyrene beads (3000 Serie Nanosphere #3100A/#3400A; ThermoFisher Scientific, Waltham, MA, USA) diluted in distilled water.

BCA protein assay: The Micro BCA Protein-Assay-Kit (ThermoFisher) was used according to the manufacturer's instructions, wherein the detection range is from 0.5 µg to 20 µg/ml. The measurements were carried out in duplicates in 96-well plates. Firstly, working reagents A, B and C were mixed in the proportion 25:24:1. The 75 µl of each standard protein solution and samples (5 x diluted with 1X PBS) were mixed with 75 µl of the working reagent to reach the total volume of 150 µl. The plates were sealed using parafilm, covered by aluminium foil and incubated at 37 °C for 2h. The absorbance was then measured using an Infinite M Plex TECAN plate reader at O.D. 562 nm and the protein concentration was determined using the standard curve.

MACSPlex Human Exosome analysis: The MACSPlex Exosome Human Kit (Miltenyi Biotech, Bergisch-Gladbach, Germany) was used according to the manufacturer's instructions. In brief, the protocol of overnight incubation at RT in a constantly rotating shaker in 1.5 ml tubes for capturing and a combination of CD63, CD81 and CD9 APC antibodies (5 µl each) for detection were applied to increase the sensitivity. The same sample volumes for the different time points were used, namely 120µl fractions (undiluted). Flow cytometric analysis was carried out on the BD Accuri TM C6 analyser with the BD Accuri software. For further analysis, background values of the control sample (PBS) and buffer (iodixanol) of each sample were subtracted from the final values. The surface marker values below the corresponding control antibody included in the kit, considered as measurement threshold, were regarded as negative. The calibration of the Flow cytometer to use MACSPlex kit was carried out according to the following four steps procedure: (i) gating a population at SSC-A/FSC-H mode; (ii) detection of 39 epitopes-associated populations (37 antibodies and 2 isotype controls) following the instruction; (iii) controlling a presence of eight populations at FL2-H/FL4-H mode and (iv) measurement of intensity at FL4-H channel for each population.

RNA isolation: RNA isolation from EV fractions was carried out using the NORGEN Total RNA Purification kit following the manufacturer's instructions. In brief, all steps were carried out at RT in an RNase-free environment according to the manufacturer's guidelines. 400µl of the fractions were divided into two aliquots in RNase-free tubes. 594µl of Buffer RL and 6 µl of Mercaptoethanol were added to every 200µl. After 10 sec vortex, 200µl of 99% Ethanol was added to every 400µl of the lysate. The final lysate was mixed well again by 10 sec vortex. Binding RNA to the column was performed by repeating the following steps: loading of 600µl of the lysate onto the column->incubation for 10 min -> centrifugation for 1 min at 4.000xg -> discarding the flow-through and reassembling the column to the collection tube. The procedure was done for the whole lysate 1,2ml volume. The washing of the column was performed by four times loading of 400µl of Wash A to the column and 1 min at 11.000xg centrifugation. The flow-through was discarded each round and the last centrifugation step was done with the highest speed for 2 min. The RNA was eluted by applying 50µl of Elution Solution A to the column and 2 min incubation with following for 1 min at 2.000xg and for 1 min at 14.000xg centrifugations.

Determination of the RNA concentration: RNA concentration measurements were carried out using a NanoDrop according to the manufacturer's instructions. In brief, the sensor of the NanoDrop was calibrated with 2µl of nuclease-free water and 2µL of elution solution to the initial blank setting. 2µl of isolated RNA of each sample was applied and measured at 260 nm twice.

cDNA-synthesis: cDNA synthesis from total isolated RNA was carried out using the M-MLV RT kit by Promega. In brief, 200 ng total RNA were used. First, 1µl of Oligo dT (100µM) was mixed with 7 µl of RNA derived from the EV-fractions, nuclease-free H₂O was added to reach a final volume of 15µl. The sample was then incubated in the Thermo Cycler for 5 min at 70 °C to denature the RNA. In the meantime, the reverse transcription mix was prepared according to the manufacturer's instruction. After denaturation, 10 µl of the mix were added to the RNA for a final volume of 25µl. For cDNA-synthesis samples were subsequently incubated in the Thermo Cycler for 60 min at 42 °C, inactivated for 15 min at 70 °C and then stored at -20 °C.

Droplet digital PCR (ddPCR) of AR-variants: cDNA from cellular RNA of DU145 cells was used as a negative control, and cDNA from cellular RNA of 22Rv1 cells as a positive control for AR-V7 and AR-FL. All probes were prepared as quadruplicates. The sequences of the labelled probes used in the ddPCR were as shown in Table 1 and the sequences of the primers were as shown in Table 2. A master-mix was prepared with the components as shown in Table 3. To generate the droplets, 20 µl of each sample were pipetted into the middle-well of a droplet generator cartridge and 70 µl of droplet generator oil into the bottom-well. The cartridge was covered with a gasket and placed into the QX100 droplet generator. 40 µl of droplet-containing emulsion located in the top-wells were carefully transferred into a filtered 96-well plate. Finally, the 96-well plate was sealed using an aluminium foil and located in the C1000 Touch Thermal Cycler to perform PCR with the settings from Table 4. After amplification, the 96-well plate was transferred to the QX100 droplet reader, read and analysed with the Quantasoft Software (1.7). All signals for the *hAR-FL* were represented by green dots and the *hAR-V7*splice variant by blue dots. Double positive results were displayed in orange colour and negatives in black.

**Table 1:**

| Probe | Sequence 5'-3' |
|---|---|
| hAR-FL (SEQ ID NO:1) | HEX-CGACCATTTCTGACAACGCCAA-Iowa Black |
| hAR-V7 (SEQ ID NO:2) | 6FAM-AAGCAACTGTGTCTGTCTGAGGT-Iowa Black |

**Table 2:**

| Primer | Sequence 5'-3' |
|---|---|
| hAR-FL_ddPCR_fwd | CCTCCAAGGACAATTACTTA (SEQ ID NO:3) |
| hAR-FL_ddPCR_rev | CACTGCCTTACACAACTC (SEQ ID NO:4) |
| hAR-V7_ddPCR_fwd | CAGGAAGAAACTTTGCTG (SEQ ID NO:5) |
| hAR-V7_ddPCR_fwd | CATCTTTATTTGTGTATTAGGC (SEQ ID NO:6) |

**Table 3:**

| 1x Reagent | Volume |
|---|---|
| 2x ddPCR supermix for probes | 10µl |
| Labeled probe hAR-FL-HEX (5 µM) | 1µl |
| Labeled probe hAR-V7-6FAM (5 µM) | 1µl |
| Primer hAR-FL fwd (90 µM) | 0.2µl |
| Primer hAR-FL rev (90 µM) | 0.2µl |
| Primer hAR-V7 fwd (90 µM) | 0.2µl |
| Primer hAR-V7 rev (90 µM) | 0.2µl |
| cDNA | 2/7µl |
| Nuclease-free H₂O | 5.2µl |

**Table 4:**

| Step | Temperature | Time |
|---|---|---|
| 1 | 95°C | 10 min |
| 2 | 95°C | 30 sec |
| 3 | 58°C (depending on primer) | 1 min |
| 4 | Go to step 2, 39x | |
| 5 | 98°C | 10 min |
| 6 | 12°C | hold |

## Claims

1. An *in vitro* method of diagnosing prostate cancer (PCa) and/or a stage thereof in a subject suspected to or known to suffer from PCa, comprising the steps of:
a) providing a plasma sample from the subject;
b) isolating from the plasma sample provided in step a) extracellular vesicles (EVs) with a size ranging from about 50 to about 400 nm and a density of about 1.10 g/ml to about 1.17 g/ml;
c) determining the levels of (i) at least one marker indicating a monocyte/ macrophage-origin, (ii) at least one marker indicating a T-cell-origin and (iii) at least one marker indicating a B-cell origin of the EVs isolated in step b);
d) determining the RNA copy number of an androgen receptor splice variant of the EVs isolated in step b); and
e) assigning PCa and/or a stage thereof to the subject, wherein, when compared to the levels of EVs of a subject with benign prostatic hyperplasia,
i. a higher copy number of an androgen receptor splice variant RNA determined in step d) is indicative of PCa; and
ii. a higher level of all of (i) to (iii) determined in step c); and a higher copy number of an androgen receptor splice variant RNA determined in step d) is indicative of therapy-resistant castration-resistant PCa (CRPC).

2. The *in vitro* method of claim 1, wherein in step c) the levels of (i) at least CD14 indicating a monocyte/ macrophage-origin, (ii) at least CD4 and CD49e indicating a T-cell-origin and (iii) at least CD19 indicating a B-cell origin of the EVs isolated in step b) are determined; and in step e) PCa and/or a stage thereof is assigned, wherein, when compared to the levels of EVs of a subject with benign prostatic hyperplasia,
(i) a higher copy number of an androgen receptor splice variant RNA determined in step d) is indicative of PCa;
(ii) a higher level of CD19 determined in step c); and a higher copy number of an androgen receptor splice variant RNA determined in step d) are indicative of hormone-sensitive PCa; and
(iii) a higher level of CD4, CD14, CD19 and CD49e determined in step c); and a higher copy number of an androgen receptor splice variant RNA determined in step d) are indicative of therapy-resistant CRPC.

3. The *in vitro* method according to claim 2, wherein in step e) (ii) a higher level of CD19 but not of CD4, CD14 and CD49e determined in step d); and a higher copy number of an androgen receptor splice variant RNA determined in step d) are indicative of hormone-sensitive PCa.

4. The *in vitro* method according to claim 2 or 3, wherein the stage of PCa is hormone-sensitive PCa, CRPC, or therapy-resistant CRPC, preferably hormone-sensitive PCa or therapy-resistant CRPC.

5. The *in vitro* method according to any one of claims 1 to 4, wherein the EVs are isolated from the plasma sample provided in step (a) by a method comprising at least the steps of (i) centrifuging the sample at a low speed and a low temperature for about 20 to 60 minutes; (ii) ultracentrifuging the supernatant from (i) at a high speed and a low temperature for at least about 30 minutes using a density gradient medium; and (iii) selecting the fractions at densities of about 1.10 g/ml to about 1.17 g/ml.

6. Use of a characteristic of extracellular vesicles (EVs) in a method of diagnosing cancer and/or a stage thereof, wherein the characteristic of the EVs is selected from the group consisting of the size, the density, the origin and combinations thereof.

7. The use according to claim 6, wherein the origin is determined using at least one surface marker indicating a specific cell type, preferably wherein the specific cell type is selected from the group consisting of cancer cells, platelets, stem cells, monocytes/ macrophages, T-cells, B-cells, dendritic cells, endothelial cells, neural cells and stromal cells.

8. The use according to claim 6 or 7, wherein the characteristic is combined with a biomarker of EVs as further characteristic of EVs, preferably wherein the biomarker of EVs is at least one biomarker selected from the group consisting of a protein, RNA, DNA, an epigenetic marker, a lipid and combinations thereof.

9. The use according to any one of claims 6 to 8, wherein the EVs are derived from a plasma sample of a subject.

10. A method of establishing a method of diagnosing cancer and/or a stage thereof, wherein the method of establishing comprises a step of analyzing a characteristic of extracellular vesicles (EVs), wherein the characteristic of the EVs is selected from the group consisting of the size, the density, the origin, and combinations thereof.

11. The method according to claim 10, wherein a biomarker as further characteristic of EVs is analyzed.

12. The method according to claim 11, wherein the biomarker is selected from the group consisting of a protein, RNA, DNA, an epigenetic marker, a lipid and combinations thereof.

13. The method according to any one of claims 10 to 12, wherein the EVs are derived from (i) a plasma sample of a subject not suffering from cancer, (ii) a plasma sample from a subject suffering from cancer and/or (iii) a plasma sample from a subject suffering from a stage of the cancer.

14. The method according to claim 13, wherein a size and/or density and/or origin and/or, if analyzed, biomarker of EVs is assigned to the EVs derived from (i) to (iii).

15. The method according to claim 14, wherein a difference between the size and/or density and/or origin and/or, if analyzed, biomarker of EVs is determined between the EVs derived from (i) to (iii).
